# FASCICULE DE BREVET EUROPEEN

(11) **EP 1 248 638 B1**
(45) Date de publication et mention de la délivrance du brevet: **08.11.2006**
(21) Numéro de dépôt: 01903859.5
(22) Date de dépôt: 03.01.2001
(51) Int. Cl.: A61K 31/727, A61P 9/10

(54) **UTILISATION DE L'ENOXAPARINE POUR LE TRAITEMENT DE L'ISCHEMIE CEREBRALE**
VERWENDUNG VON ENOXAPARIN ZUR BEHANDLUNG DER ZEREBRALEN ISCHÄMIE
USE OF ENOXAPARIN FOR THE TREATMENT OF CEREBRAL ISCHEMIA

(30) Priorité: 06.01.2000 FR 0000137
(43) Date de publication de la demande: 16.10.2002
(73) Titulaire: Aventis Pharma S.A., 92160 Antony (FR)
(72) Inventeur: Mary, Véronique, 91130 Ris Orangis (FR); Stutzmann, Jean-Marie, 94440 Villecresnes (FR); Uzan, André, 75116 Paris (FR); Wahl, Florence, 75004 Paris (FR)
(74) Mandataire: Rousseau, Pierrick Edouard
(86) Numéro de dépôt international: PCT/FR2001/000014
(87) Numéro de publication internationale: WO 2001/049298

(56) Documents cités:
- WO-A-94/08595
- US-A- 5 767 269
- LI ET AL.: "Posttreatment with low molecular weight heparin reduces brain edema and infarct volume in rats subjected to thrombotic middle cerebral arety occlusion" BRAIN RES., vol. 801, no. 1-2, - 1998 pages 220-223, XP000965075
- HILLBOM ET AL.: "Comparison of the efficacy of the low molecular weight heparin enoxaparin with ubfractionated heparin in the prevention of deep vein thrombosis in patients with acute ischemic stroke" BLOOD, vol. 94, no. 10sup1,pt1, 15 novembre 1999 (1999-11-15), page 183a XP000961033
- SAMANA ET AL.: "Acute ischemic stroke and heparin treatments" THROMB. HAEMOSTASIS, vol. 78, no. 1, 1997, pages 173-179, XP000964515
- GORDON ET AL.: "low molecular weight heparins and heparinoids and their use in acute or progressing ischeminc stroke" CLIN. NEUROPHARMACOL., vol. 13, no. 6, 1990, pages 522-544, XP000965540
- KAY ET AL.: "low molecular weight heparin for the treatment of acute ischemic stroke" NEW ENGLAND J. MED., vol. 333, no. 24, 1995, pages 1588-1593, XP000965497
- BATH ET AL.: "low molecular weight heparin in acute stroke" EXPERT OPINION INVEST. DRUGS, vol. 7, no. 8, 1998, pages 1323-1330, XP000965050
- HARENBERG ET AL.: "enoxaparin is superior to unfractionated heparin in the prevention of thromboembolic events in medical patients at increased thromboembolic risk" BLOOD, vol. 94, no. 10pt1.suppl.1, 1999, page 399a XP000961034
- TAIST INVESTIGATORS: "Tinzaparin in acute ischemic stroke trial (TAIST)" STROKE, vol. 30, no. 1, janvier 1999 (1999-01), page 263 XP000965045
- CHEMICAL ABSTRACTS, vol. 127, no. 24, 1997 Columbus, Ohio, US; abstract no. 326219z, page 54; XP002154060 & XIAO ET AL.: "low molecular weight heparin in treatment of acute cerebral infarction" GUANGDONG YIXUE, vol. 18, no. 7, - 1997 pages 449-450,
- CHEMICAL ABSTRACTS, vol. 130, no. 1, 1999 Columbus, Ohio, US; abstract no. 336b, page 44; XP002154061 & ZHENG ET AL.: "clinical study on low molecular weight heparin (LMWH) for treatment of acute cerebral infarction" ZHEJIANG YIKE DAXUE XUEBAO, vol. 27, no. 3, 1998, pages 114-116,
- DAHL ET AL.: "Dalteparin in acute ischemic cerebrovascular disease: a safety study" CEREBROVASCULAR DISEASES, vol. 7, no. 1, 1997, pages 28-33, XP000965430
- PRINS ET AL.: "Prophylaxis of deep venous thrombosis with a low molecular weight heparin (Kabi 2165 / Fragmin) in stroke patients" HAEMOSTASIS, vol. 19, no. 5, 1989, pages 245-250, XP000965043
- KAY ET AL.: "Pilto study of low molecular weight heparin in the treatment of acute ischemic stroke" STROKE, vol. 25, no. 3, mars 1994 (1994-03), pages 684-685, XP000965046
- RYU ET AL.: "Heparin reduces neurological impairment afetr cerebral air embolism in the rabbit" STROKE, vol. 27, no. 2, 1996, pages 303-310, XP000965482
- STULLKEN ET AL.: "The effects of heparin on recovery from ischemic brain infuries in cats" ANESTH. ANALG., vol. 55, no. 5, 1976, pages 683-687, XP000965059
- XANAKA ET AL.: "Reduction of brain injury using heparin to inhibit leukocyte accumulation in a rat model of transient focal cerebral ischemia. II. Dose-response effect and the therapeutic window" J. NEUROSURG., vol. 85, no. 6, 1996, pages 1108-1112, XP000965407
- QUATERMAIN ET AL.: "Enoxaparin, a low molecular weight heparin decreases infarct size and improves sensorimotor function in a rat model of focal cerebral ischemia" NEUROSCI. LETTERS, vol. 288, no. 2, 14 juillet 2000 (2000-07-14), pages 155-158, XP001034868

## Description

La présente invention concerne une nouvelle application thérapeutique de l'énoxaparine.

L'énoxaparine est une héparine de bas poids moléculaire (BATH et coll., Expert Opinion Invest. Drugs, vol. 7, 8, 1323-1330 (1998) ; GORDON et coll., Clin. Neuropharmacol., vol. 13, 6, 522-544 (1990)). L'énoxaparine (Lovenox^{R}, Clexane^{R}) est commercialisée pour le traitement prophylactique de la maladie thromboembolique veineuse en chirurgie à risque modéré ou élevé, la prévention de la coagulation du circuit de circulation extra-corporelle au cours de l'hémodialyse, le traitement des thromboses veineuses profondes constituées et en association avec l'aspirine pour le traitement de l'angor instable et de l'infarctus du myocarde sans onde Q à la phase aiguë. L'énoxaparine est également utile dans la prévention et/ou le traitement du trauma du système nerveux central (WO98/53833) et des oedèmes cérébraux (WO98/53834).

Des héparines de bas poids moléculaire ont été testées dans la prévention et/ou le traitement des thromboses veineuses profondes chez des patients présentant une ischémie cérébrale mais aucun effet sur l'ischémie n'a été montré (A. ELIAS et coll.,La Revue de Médecine Interne, 1, tome XI, 95-98 (1990); HILLBOM et coll., Blood, vol. 94, 10, 183a (1999); MH. PRINS et coll., Haemostasis, 19, 245-250 (1989); AGG. TURPIE et coll., The Lancet, 523-526 (1987)). Une héparine de bas poids moléculaire a été décrite comme réduisant l'oedème cérébral et la lésion chez des rats soumis à un modèle d'ischémie (LI et coll., Brain Res., vol. 801, 1-2, 220-223 (1998)). Une héparine de bas poids moléculaire a été testé dans le cadre d'une étude clinique pour le traitement du stroke ischémique aigu (ZHENG et coll., Chemical Abstracts, vol.130, 1, 44 (1999)). L'énoxaparine a été décrite comme étant supérieure à l'héparine dans la prévention de risques thromboemboliques chez des patients présentant une maladie respiratoire sévère ou un stroke ischémique aigu, mais aucun effet sur l'ischémie n'a été montré (HARENBERG et coll., Blood, vol. 94, 10, 399a (1999)).

Il a maintenant été trouvé que l'énoxaparine permet de réduire les séquelles ischémiques cérébrales et peut ainsi être utilisée pour le traitement des ischémies cérébrales.

Cette nouvelle utilisation thérapeutique a été déterminée chez le rat selon le protocole suivant :

Des rats mâles Sprague Dawley (230-250g Iffa Credo) sont nourris et abreuvés ad libitum et maintenus dans un cycle lumière-obscurité de 12 heures. La chirurgie est réalisée sous halothane (1,4% dans un mélange de 70% N₂O/30% O₂). Pendant l'anesthésie, la normothermie est maintenue en plaçant le rat sous une couverture thermostatée. L'artère carotide commune gauche est isolée et une ligature lâche est mise en place. L'artère cérébrale moyenne gauche est exposée via une craniotomie sous-temporale et un microclamp est placé dans la région proximale de l'artère, immédiatement suivi par la ligature de l'artère carotide. Deux heures plus tard, les animaux sont réanesthésiés et la circulation cérébrale est rétablie en enlevant le clamp de l'artère cérébrale moyenne et la ligature de l'artère carotide. Les rats sont alors placés dans leur cage dans une pièce thermostatée à 26-28°C.

48 heures après la chirurgie un examen neurologique est réalisé pour chaque rat par un examinateur ignorant du traitement. L'échelle neurologique utilisée est décrite dans le tableau 1.

**TABLEAU 1**

| Item | | Normal | Déficit |
|---|---|---|---|
| Réflexe d'aggrippement | patte antérieure droite | 1 | 0 |
| Réaction de placement | | | |
| Perte d'appui | patte antérieure droite | 1 | 0 |
| | patte postérieure droite | 1 | 0 |
| réflexe de redressement | | | |
| rotation | coté droit | 1 | 0 |
| | coté gauche | 1 | 0 |
| Postures anormales | | Absent | Présent |
| Flexion patte antérieure droite | | 1 | 0 |
| Torsion du corps | | 1 | 0 |
| | | | |
| Score neurologique global | | 7 | 0 |

Après l'examen neurologique, les rats sont euthanasiés et leurs cerveaux prélevés. Des coupes sériées de 1,5 mm d'épaisseur sont préparées et colorées par le chlorure de 2,3,5-triphenyltétrazolium (TTC) à 2%. Après 24 heures de post-fixation dans une solution de formaldéhyde à 10%, les aires de lésion (infarct cérébral) sont mesurées aux niveaux cortical et striatal; les volumes sont calculés par intégration des surfaces lésées. Les valeurs sont exprimées en mm³ (moyenne ± E.S.M). L'analyse statistique est effectuée par un test de Mann-Whitney ou par un test de Kruskal-Wallis pour analyse de variance non-paramétrique suivie d'un test de Dunn pour comparaison entre groupes (*: p<0,05 , **: p<0,01, ***: p<0,001 vs groupe contrôle).

Dans l'étude 1, l'énoxaparine est administrée à 12 rats à 1,5 mg/kg iv, 2 heures et 24 heures après le début de l'ischémie. Un groupe contrôle de 10 rats ne reçoit que le véhicule (Soluté physiologique de chlorure de sodium à 0,9%) suivant le même protocole.

Dans l'étude 2, la fenêtre d'opportunité thérapeutique de l'énoxaparine est explorée. Le traitement débute 5 heures après l'ischémie suivi par une seconde administration à 24 heures. Cette étude consiste en un effet-dose d'énoxaparine sur les lésions cérébrales. Les doses étudiées sont 0,5; 1 et 1,5 mg/kg iv (9-10 rats par groupe). Un groupe contrôle de 11 rats ne reçoit que le véhicule (Soluté physiologique de chlorure de sodium à 0,9%).

Dans l'étude 3, l'énoxaparine est administrée à 10 rats à 1,5 mg/kg iv, 5 et 24 heures après le début de l'ischémie. Un groupe contrôle de 13 rats ne reçoit que le véhicule (Soluté physiologique de chlorure de sodium à 0,9%).

Dans l'étude 4, le protocole est le même que dans les autres études mais l'artère cérébrale moyenne gauche est cautérisée de façon permanente et on ne pratique pas d'occlusion de l'artère carotide gauche. L'énoxaparine est administrée à 13 rats à 1,5 mg/kg iv, 5 et 24 heures après le début de l'ischémie. Un groupe contrôle de 13 rats ne reçoit que le véhicule (Soluté physiologique de chlorure de sodium à 0,9%).

Les résultats obtenus sont mentionnés dans le tableau 2.

**TABLEAU 2**

| ETUDES | | LESION CORTICALE (mm3) | SCORE NEUROLOGIQUE échelle de 7 points |
|---|---|---|---|
| Etude 1 | groupe contrôle | 186 ± 18 | 1,7 ± 0,3 |
| | groupe énoxaparine | | |
| | 2x1,5 mg/kg iv | 131 ± 13* | 3,1 ± 0,2** |
| Etude 2 | groupe contrôle | 203 ± 12 | |
| | groupes énoxaparine | | |
| | 2x0,5 mg/kg iv | 164 ±15 | |
| | 2x1 mg/kg iv | 142 ± 24* | |
| | 2x1,5 mg/kg iv | 129 ± 17* | |
| Etude 3 | groupe contrôle | 195 ± 12 | 1,8 ± 0,3 |
| | groupe énoxaparine | | |
| | 2x1,5 mg/kg iv | 129 ± 16** | 3,4 ± 0,3*** |
| Etude 4 | groupe contrôle | 137 ± 23 | 1,7 ± 0,2 |
| | groupe énoxaparine | | |
| | 2x1,5 mg/kg iv | 71 ± 13* | 2,9 ± 0,3** |

Ces résultats démontrent que
- dans l'étude 1, l'énoxaparine améliore significativement le score neurologique 48 heures après l'ischémie cérébrale et, de plus, réduit significativement la lésion corticale de 30%,
- dans l'étude 2, l'énoxaparine réduit la lésion corticale de 30 % (2x1 mg/kg) et 36 % (2x1,5 mg/kg),
- dans l'étude. 3, l'énoxaparine améliore significativement le score neurologique et réduit la lésion corticale de 34 %,
- dans l'étude 4, l'énoxaparine améliore significativement le score neurologique et réduit la lésion corticale de 49%.

Aucun problème de saignement n'a été rencontré lors de ces études.

Les médicaments sont constitués par l'énoxaparine sous forme d'une composition dans laquelle il est associé à tout autre produit pharmaceutiquement compatible, pouvant être inerte ou physiologiquement actif. Les médicaments utilisés selon l'invention peuvent être employés, de préférence, par voie intraveineuse ou sous-cutanée.

Les compositions stériles pour administration intraveineuse ou sous-cutanée, sont généralement des solutions aqueuses. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation. Elles peuvent également être préparées sous forme de compositions solides stériles qui peuvent être dissoutes au moment de l'emploi dans de l'eau stérile ou tout autre milieu stérile injectable.

Comme exemple d'une composition, on peut dissoudre 20 mg d'énoxaparine dans de l'eau distillée en quantité suffisante pour préparer 0,2 ml de solution.

Les doses dépendent de l'effet recherché, de la durée du traitement et de la voie d'administration utilisée; elles sont généralement comprises entre 0,2 mg et 4 mg/kg par jour par voie sous-cutanée soit 14 à 280 mg par jour pour un adulte avec des doses unitaires allant de 5 à 280 mg.

D'une façon générale, le médecin déterminera la posologie appropriée en fonction de l'âge, du poids et de tous les autres facteurs propres au sujet à traiter.

La présente invention concerne l'utilisation de l'énoxaparine pour la préparation d'un médicament destiné à la réduction des séquelles ischémiques cérébrales, définie par une amélioration du score neurologique et/ou une diminution de la taille des lésions corticales, dans le traitement l'ischémie cérébrale.

## Revendications

1. Utilisation de l'énoxaparine pour la préparation d'un médicament destiné à la réduction des séquelles ischémiques cérébrales, définie par une amélioration du score neurologique et /ou une diminution de la taille des lésions corticales, dans le traitement l'ischémie cérébrale.

2. Utilisation selon la revendication 1 pour la préparation de médicaments contenant 5 à 280 mg d'énoxaparine.

## Claims

1. Use of enoxaparin for the preparation of a medicament intended to reduce cerebral ischaemia after-effects, defined by an improvement in the neurological score and/or a decrease in the size of the cortical lesions, in the treatment of cerebral ischaemia.

2. Use according to Claim 1, for the preparation of medicaments containing 5 to 280 mg of enoxaparin.

## Patentansprüche

1. Verwendung von Enoxaparin zur Herstellung eines Arzneimittels zur Verringerung von Folgeerscheinungen von zerebraler Ischämie, definiert durch eine Verbesserung des neurologischen Scores und/oder eine Verringerung der Größe von kortikalen Läsionen, bei der Behandlung von zerebraler Ischämie.

2. Verwendung nach Anspruch 1 zur Herstellung von Arzneimitteln, die 5 bis 280 mg Enoxaparin enthalten.
